# EUROPÄISCHE PATENTSCHRIFT

(11) **EP 0 734 234 B1**
(45) Veröffentlichungstag und Bekanntmachung des Hinweises auf die Patenterteilung: **21.04.1999**
(21) Anmeldenummer: 95901452.3
(22) Anmeldetag: 29.11.1994
(51) Int. Cl.: A61C 1/00

(54) **VORRICHTUNG ZUM ENTFERNEN VON ABLAGERUNGEN AUF EINEM ZAHN**
DEVICE FOR REMOVING DEPOSITS FROM TEETH
DISPOSITIF D'ELIMINATION DES DEPOTS SUR LES DENTS

(30) Priorität: 17.12.1993 DE 4343218
(43) Veröffentlichungstag der Anmeldung: 02.10.1996
(73) Patentinhaber: RECHMANN, Peter, 40627 Düsseldorf (DE)
(72) Erfinder: RECHMANN, Peter, 40627 Düsseldorf (DE)
(74) Vertreter: Dallmeyer, Georg, Dipl.-Ing.
(86) Internationale Anmeldenummer: EP9403950
(87) Internationale Veröffentlichungsnummer: WO9516404

(56) Entgegenhaltungen:
- EP-A- 0 515 983
- DE-A- 3 841 503
- DE-A- 4 015 066
- DE-C- 4 014 303
- US-A- 4 950 267
- US-A- 5 246 436

## Beschreibung

Die Erfindung betrifft eine Vorrichtung zum Entfernen von mikrobieller Plaque, Zahnstein und Konkrementen auf einem Zahn nach dem Oberbegriff des Anspruchs 1.

Zahnstein und Konkremente werden üblicherweise mit Ultraschallinstrumenten und Handinstrumenten mechanisch entfernt. Bei Verwendung von Ultraschallinstrumenten kommt es durch die Schwingungsbewegungen der Instrumentenspitze zu einem Absprengen der kalzifizierten Strukturen. Kleine mineralisierte Teilbereiche bleiben aber dabei auf der Zahnoberfläche übrig und dienen der schnellen Neuanhaftung von Plaque und der anschließenden Auskristallisierung zu weiterem Zahnstein. Zahnstein kann vollständig durch zusätzliche Anwendung von Handinstrumenten entfernt werden. Hierzu dienen Scaler etc., die von Hand mit Druck über die Zahnoberfläche gezogen werden müssen. Nachteilig ist, daß die Instrumentenspitze bei der Ultraschallentfernung durch ihre Schwingungen zur Zerstörung von Zahnhartsubstanz führen kann. Mit den Handinstrumenten kommt es auch zu Schäden an der Zahnhartsubstanz, insbesondere wenn das Instrument nicht parallel zur Zahnoberfläche geführt wird. Derartige Beschädigungen sind zwangsläufig, da es nicht möglich ist, an allen Stellen des Zahnes tangential zu der Zahnoberfläche zu schaben.

Im Bereich des Taschenbodens eines Zahnes wird die vollständige Konkremententfernung schon deswegen schwierig, weil das schmalste Handinstrument im Vergleich zur Weite der Zahnfleischtasche zwischen Zahn und Zahnfleisch sehr groß ist. Damit alle Konkremente entfernt werden können, muß man mit dem Instrument unter die Konkremente gelangen und löst dabei die obersten Fasern des Zahnhalteapparates. Für tiefere Zahnfleischtaschen, beispielsweise über 4 mm Tiefe, insbesondere bei mehrwurzligen Zähnen, wird daher die Konkremententfernung unter Sicht, d.h. nach chirurgischer Aufklappung des Zahnfleisches durchgeführt.

Aus der Druckschrift Aoki, A. et. al.: Basic Studies on the Application of Er:YAG Laser to Scaling, Proceedings of the ISLD ist eine gattungsgemäße Vorrichtung bekannt. Third International Congress on Laser in Dentistry, Salt Lake City, Utah, 1992) einen Er: YAG-Laser zur Entfernung von Konkrementen im subgingivalen Bereich (Wurzelglätten) zu verwenden. Die Laserlichtquelle mit einer Wellenlänge von 2,94 µm wird mit einer maximalen Frequenz von 10 Hz und einer Pulsdauer von 200 µs betrieben. Die Effizienz dieses Lasers ist unterhalb einer Energie von 10 mJ/Impuls sehr gering. Erst bei höheren Energien zwischen 20 bis 30 mJ/Impuls bei 10 Hz Impulsfrequenz ist eine akzeptable Effizienz erreichbar mit dem Nachteil, daß die Zementschicht an der Zahnwurzel beschädigt wird. Bei Energien oberhalb von 30 mJ/Impuls sind die Beschädigungen der Zementschicht sogar gravierend. Ein weiterer Nachteil besteht in der Gefahr der Schädigung des Zahnfleisches.

Der bekannte Laser hat eine hohe Absorptionscharakteristik für Wasser. Dies führt dazu, daß durch Richten des Laserlichtstrahls auf die Taschenwand oder den Taschenboden der Zahnfleischtasche, welches sich durch die engen anatomischen Verhältnisse immer ereignen kann, ein unkontrollierter Abtrag dieser Gewebestrukturen auch bei Verwendung niedrigster Laserflußdichten erfolgen kann.

Aus DE-C-40 14 303 ist eine Vorrichtung zum selektiven Entfernen von Zahnkaries mit Laserlicht bekannt. Sie weist die strukturellen Merkmale des Oberbegriffs von Anspruch 1 auf. Die bekannte Vorrichtung macht sich zunutzen, daß für gesundes Dentin und Karies ein unterschiedlicher Ablationsdruck entsteht, und daß aufgrund dieser Unterschiede bei Auswahl geeigneter Betriebsparameter für den Laser ein selektives Entfernen von kariösen Dentinsubstanzen möglich ist.

Aus der EP-A- 515 983 ist ein zahnmedizinisches Handstück bekannt, bei dem der Kühlflüssigkeitsstrahl koaxial mit dem Laserlicht austritt. Der in Verbindung mit dem Handstück beschriebene Laser emittiert Laserlicht einer Wellenlänge, das gesundes Zahnmaterial angreift. Desweiteren ist das Handstück nicht zum Einsatz in Zahnfleischtaschen geeignet.

Aus der Veröffentlichung "Zahnärztliche Praxis, Heft 2, 1990, Seiten 75/76" ist die Verwendung eines Excimerlasers zum Säubern von Wurzelkanälen und zum Abtragen von Zahnschmelz bekannt. Die Wellenlänge des Laserlichtes beträgt 308 nm. Dieser Laser wird mit hoher Energiedichte betrieben, so daß er wie ein Bohrwerkzeug, das Zahnschmelz abträgt, verwendet werden kann.

Aus der DE 39 36 714 A1 ist eine Munddusche beschrieben, bei der in einer Schlauchleitung zwischen der Flüssigkeitspumpe und einem Handstück zwei flexible Lichtleiter angeordnet sind, die dazu dienen, Schaltfunktionen an den Elektromotor der Flüssigkeitspumpe stromlos zu übertragen.

Der Erfindung liegt die Aufgabe zugrunde, eine Vorrichtung zum Entfernen von Plaque, Zahnstein und Konkrementen zu schaffen, die selektiv und mit hoher Effizienz die zu entfernenden Substanzen abträgt, ohne die Zahnoberfläche oder das Zahnfleisch zu schädigen.

Zur Lösung dieser Aufgabe dienen die Merkmale des Anspruchs 1.

Die Erfindung sieht in vorteilhafter Weise vor, daß die Laserlichtquelle zur Entfernung der Ablagerungen und von bakteriell infiziertem Wurzelzement einen Laserlichtstrahl mit einer dem Absorbtionsverhalten der zu entfernenden Substanzen selektiv angepaßten Wellenlänge von 300 bis 600 nm, vorzugsweise von 320 bis 410 nm, und mit einer Energiedichte von 0,5 bis 10 J/cm² emittiert, die das Zahnfleisch und die gesunde Zahnsubstanz nicht schädigt.

Es ist dabei vorgesehen, daß der Laserlichtstrahl in einen Flüssigkeitsstrahl eingekoppelt wird und koaxial mit dem Flüssigkeitsstrahl aus dem Applikator austritt. Der Laserlichtstrahl folgt in diesem Fall exakt dem Verlauf des Flüssigkeitsstrahls, der als Verlängerung des Lichtleiters dient. Der Arbeitspunkt des Laserlichtstrahls ist damit identisch mit dem Auftreffpunkt des Flüssigkeitsstrahls, wodurch erreicht wird, das der Flüssigkeitsstrahl die entfernten Ablagerungen gleich fortspülen kann, und daß die bearbeiteten Stellen gekühlt werden. Die Laserlichtenergiedichte bleibt über einen bestimmten Flüssigkeitsstrahlabschnitt konstant. Es ist daher möglich, den Applikator auch im Abstand von dem Arbeitspunkt einzusetzen. Dies ist für den supragingivalen Bereich von Interesse, während in der Zahnfleischtasche aus Platzgründen der Applikator mit der Austrittsdüse für den Flüssigkeitsstrahl und den Laserlichtstrahl direkt auf die zu bearbeitende Stelle aufgesetzt wird. Vorteilhaft ist dabei, daß es nicht zu einem Abbrand des Lichtleiterendes kommen kann.

Die Auswahl der Laserparameterkombination gewährleistet eine hohe Effektivität der Ablation von mikrobieller Plaque, Zahnstein und Konkrementen, ohne daß das Gewebe der Zahnfleischtasche oder die gesunde Zahnhartsubstanz, z.B. Schmelz unter supragingivalem Zahnstein, sowie Schmelz, gesunder Wurzelzement und gesundes Wurzeldentin unter subgingivalem Zahnstein, unnötig geschädigt wird. Die Tascheninnenwand sowie der parodontale Halteapparat wird nicht geschädigt, wenn der Laserlichtstrahl auf die Taschenwand oder auf den Taschenboden trifft. Die Energieflußdichten sind hierfür ausreichend niedrig gewählt, insbesondere im Hinblick auf das Absorptionsverhalten der abzutragenden Substanzen bei der genannten Wellenlänge des Laserlichtes. Es ist somit möglich, selektiv die unerwünschten Ablagerungen auf Zähnen zu entfernen, ohne das Zahnfleisch oder die gesunde Zahnsubstanz zu schädigen. Des weiteren ist es möglich, bakteriell infizierten Wurzelzement zu entfernen, wodurch eine Regeneration der Zahnhaltestrukturen eingeleitet werden kann.

Bei einem Durchmesser von ca. 1 bis 1,5 mm kann der Applikator in tiefe Zahntaschen eingeführt werden. Mit Hilfe des Fluidstrahls kann das Laserlicht bis in die tiefsten Bereiche der Zahntasche vordringen.

Die Pulsdauer eines Laserlichtimpulses oder eines gütegeschalteten Spikeimpulses kann weniger als 10 µs, vorzugsweise zwischen 5 ns und 5 µs betragen. Die Impulslänge des Laserlichtes sollte 10 µs nicht überschreiten, da sonst eine thermische Schädigung des angrenzenden Gewebes nicht ausgeschlossen werden kann. Bevorzugt sind Impulslängen im Bereich zwischen 50 ns und 300 ns. Es ist auch möglich die Impulslänge des Laserlichtes in den genannten Bereichen variabel einstellbar zu halten.

Besonders bevorzugt sind Impulslängen in dem Bereich zwischen 150 und 250 ns.

Die Laserlichtimpule können auch im freilaufenden Betrieb mit einer nominellen Pulsdauer von mehreren 100 µs als Folge von Spikeimpulsen bereitgestellt werden.

Die Pulswiederholungsfrequenz der Laserlichtimpulse der Laserlichtquelle liegt im Bereich zwischen 10 und 200 Hz, vorzugsweise zwischen 15 und 150 Hz.

Dabei kann jede Impulskette des Laserlichtimpulses 2 bis 30 gütegeschaltete Spikeimpulse enthalten, von denen jeder eine Energiedichte im beanspruchten Bereich aufweist.

Der Arbeitspunkt des Laserlichtstrahls kann mit einem gerichteten Flüssigkeitsstrahl gekühlt werden.

Es kann vorgesehen sein, daß der Applikator an seinem freien Ende eine seitlich zur Längsachse des Applikators verlaufende Austrittsöffnung für den Laserlichtstrahl aufweist. Bei einer seitlichen Austrittsöffnung für den Laserlichtstrahl bzw. für den Flüssigkeitsstrahl mit eingekoppeltem Laserlichtstrahl ist ein Abtrag in der Enge der Zahnfleischtasche einfacher möglich. Die seitliche Austrittsöffnung kann beispielsweise durch entsprechende Krümmung des den Lichtleiter verlängernden Flüssigkeitskanals realisiert werden.

Der Applikator kann mit einem akustischen Sensor versehen sein. Der akustische Sensor dient zur Kontrolle des Arbeitsfortschrittes, da solange Ablagerungen vorhanden sind, das Abtragen aufgrund des Ablationsdrucks ein Geräusch erzeugt. Sobald keine Ablagerungen mehr vorhanden sind, sinkt der Geräuschpegel erkennbar, der akustisch mit Sensoren überwacht werden kann.

Zur Kontrolle des Arbeitserfolges kann auch eine Kontrolleinrichtung mit einem optischen Sensor vorgesehen sein. Diese Kontrolleinrichtung kann separat in einem weiteren Handstück oder an dem Applikator angeordnet sein.

Bei einem bevorzugten Ausführungsbeispiel ist vorgesehen, daß der optische Sensor mit einer Lochblende versehen ist. Eine Lochblende hat den Vorteil, mit einer erheblich geringeren Dimension im Vergleich zu einer Linse herstellbar zu sein. Da aufgrund des Lasers sich eine ausreichende Lichtintensität im Arbeitsbereich des Applikators befindet, ist eine Lochblende völlig ausreichend, um ein qualitätsmäßig gutes Bild des Arbeitsbereiches zu erzeugen.

Zwischen dem optischen Sensor und der Lochblende ist vorzugsweise ein Bildlichtleiter zwischengeschaltet. Ein derartiger Bildlichtleiter besteht aus einer Vielzahl gebündelter, parallel verlaufender Lichtleiter. Der Bildlichtleiter erlaubt es, den optischen Sensor in Distanz von der Zahnfleischtasche zu halten.

Die Lochblende des optischen Sensors kann flüssigkeitsdurchströmt sein. Dabei besteht die Möglichkeit, daß die Flüssigkeit durch die Lochblende selbst und/oder durch ihren Randbereich hindurchgeführt wird. Dabei wird angestrebt, daß der die Lochblende enthaltende optische Kopf sich stets in Flüssigkeit befindet. Wenn die Lochblende selbst flüssigkeitsdurchströmt ist, wird in vorteilhafter Weise verhindert, daß sie sich durch die von dem Applikator abgetragenen Ablagerungen zusetzen kann.

Bei einem besonders bevorzugten Ausführungsbeispiel ist ein akustischer Sensor dadurch in den Applikator integriert, daß der aus dem Applikator austretende laserlichtleitende Flüssigkeitsstrahl entgegen der Durchflußrichtung der Flüssigkeit den im Arbeitspunkt des Laserlichtes entstehenden Körperschall an einen akustischen Sensor überträgt. Dieses Körperschallsignal hat einen sehr niedrigen Pegel, wenn alle Ablagerungen entfernt sind und einen entsprechend höheren Pegel, wenn noch Ablagerungen abgetragen werden.

Im folgenden wird unter Bezugnahme auf die Zeichnungen ein Ausführungsbeispiel der Erfindung näher erläutert.

Es zeigen:
- Fig. 1: ein Zahn mit einem in die Zahnfleischtasche eingeführten Instrument,
- Fig. 2: den Arbeitsbereich der erfindungsgemäßen Vorrichtung auf der Zahnoberfläche,
- Fig. 3: ein Absorptionsdiagramm von Bakterien,
- Fig. 4: ein Absorptionsdiagramm von Wasser,
- Fig. 5: eine schematische Darstellung des Funktionsprinzips eines erfindungsgemäßen Applikators,
- Fig. 6: eine prinzipielle Darstellung eines zweiten Ausführungsbeispiels eines Applikators mit seitlichen Strahlaustritt,
- Fig. 7: die seitliche Austrittsöffnung eines dritten Ausführungsbeispiels des Applikators mit ovalem Querschnitt,
- Fig. 8: ein optisches Kontrollinstrument,
- Fign.9 und 10: Schnitte entlang der Linie IX-IX bzw. X-X in Fig. 8, und
- Fig. 11: ein zweites Ausführungsbeispiel eines optischen Kontrollinstrumentes.

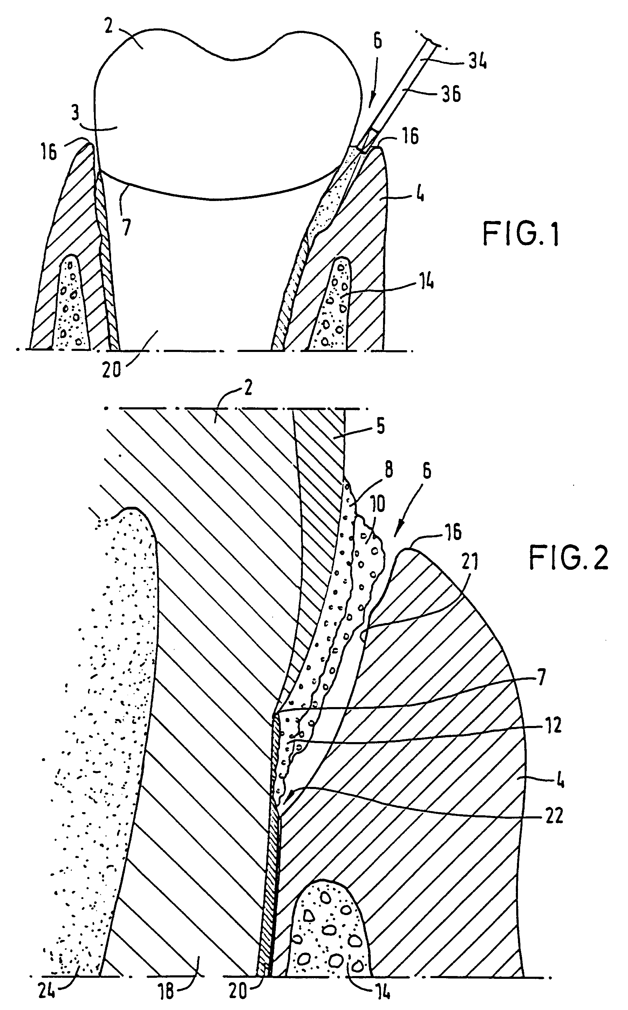

Fig. 1 zeigt einen in einem Kiefer 14 gehaltenen Zahn 2.

Auf der linken Seite des Zahnes 2 sind gesunde parodontale Verhältnisse dargestellt. Die Anheftungslinie des Zahnfleisches 4 liegt im gesunden Zustand an der Grenzlinie 7 zwischen der Zahnkrone 3 und dem Wurzelzement 20. Der obere Zahnfleischrand 16 liegt 0,5 bis 2 mm oberhalb dieser Grenzlinie 7, so daß eine natürliche Zahnfleischtasche 6 von 0,5 bis 2 mm vorliegt. Auf der rechten Seite ist ein erkrankter Zustand dargestellt. Der Taschenboden liegt aufgrund von entzündlichen Prozessen nicht im Bereich der Grenzlinie 7, sondern es hat sich eine vertiefte Zahnfleischtasche 6 gebildet. Das Zahnfleisch 4 ist jetzt weiter unterhalb der Grenzlinie 7 am Zahn 2 angeheftet.

Ablagerungen auf Zähnen treten oberhalb und unterhalb des oberen Zahnfleischrandes auf und bestehen im wesentlichen aus Zahnstein 8, mikrobieller Plaque 10 und Konkrementen 12. Bei entzündeter Zahnfleischtasche 6 und entzündetem Zahnfleisch 4 bildet sich auch die stützende Struktur des Kieferknochens 14 zurück, so daß es bei fortgeschrittener Parodontose zunächst zu einer Zahnlockerung und schließlich zu einem Zahnausfall kommt.

Der Zahn 2 weist eine Zahnkrone 3 auf, die mit einer Schmelzschicht 5 überzogen ist. Die Schmelzschicht 5 geht an einer Grenzlinie 7, die geringfügig unterhalb des oberen Zahnfleischrandes 16 verläuft, in die Wurzelzementschicht 20 über.

Mikrobielle Plaque 10 besteht aus einer bakteriellen Aggregation auf Zähnen 2, die nicht mineralisiert ist und Bakterien, Leukozyten, abgeschilferten orale Epithelzellen, Blutzellen und Nahrungsrestbestandteile enthält. In 1 mm³ Plaque sind ca. 10⁸ Bakterien enthalten. Die intermikrobielle Matrix (25 Volumen %) besteht aus Proteinen, Karbohydraten und Lipiden. Diese Stoffe stammen aus dem Bakterienstoffwechsel, dem Speichel und dem Gewebeexsudat, nämlich dem Taschenexsudat. Es kann zwischen supra- und subgingivaler Plaque unterschieden werden. Als supragingival bezeichnet man die Region oberhalb des Zahnfleischrandes 16, also den sichtbaren Zahnbereich, während subgingival den Bereich in der Zahnfleischtasche 6 kennzeichnet.

Die Zahnfleischtasche 6 kann wie folgt definiert werden:

Nach oben ist sie entweder offen oder durch supragingivale Plaque bzw. Zahnstein 8 verschlossen. Die Innenseiten der Zahnfleischtasche 6 bestehen aus einem Taschenepithel, das ggf. entzündliche Proliferationen aufweist. Zahnseitig befindet sich auf dem Wurzeldentin 18 eine Wurzelzementschicht 20, die teilweise von Bakterien bzw. Bakterientoxinen durchsetzt sein kann. Auf dieser Wurzelzementschicht 20 kann eine Konkrementschicht 12 und eine Plaqueschicht 10 folgen. Nach unten ist die Zahnfleischtasche durch den Taschenboden 22, Bindegewebsfasern und einer Epithelschicht begrenzt. Bei fortschreitender Entzündung der Zahnfleischtasche bei Parodontose verlagert sich der Taschenboden weiter nach unten.

Zahnstein 8 besteht aus mineralisierter Plaque, nämlich aus anorganischen Salzen mit den Hauptelementen Ca/P. Subgingivaler Zahnstein wird auch als Konkrement 12 bezeichnet, wobei die Konkremente 12 vermehrt Blutfarbstoffe enthalten und daher hinsichtlich der Färbung eher dunkel bis schwarz eingefärbt sind. Die schwarze Farbe ist zum Teil auf unlösliches Eisensulfid zurückzuführen, das aus bakteriellen Schwefelwasserstoff und Speicheleisen gebildet wird. Supragingivaler Zahnstein 8 ist eher weißlichgelb bis dunkelgelb.

Fig. 2 zeigt einen Schnitt im Bereich einer entzündlichen Zahnfleischtasche 6. Neben dem Wurzeldentin 18 ist der Hohlraum des Zahnes 2 für die Pulpa 24 dargestellt.

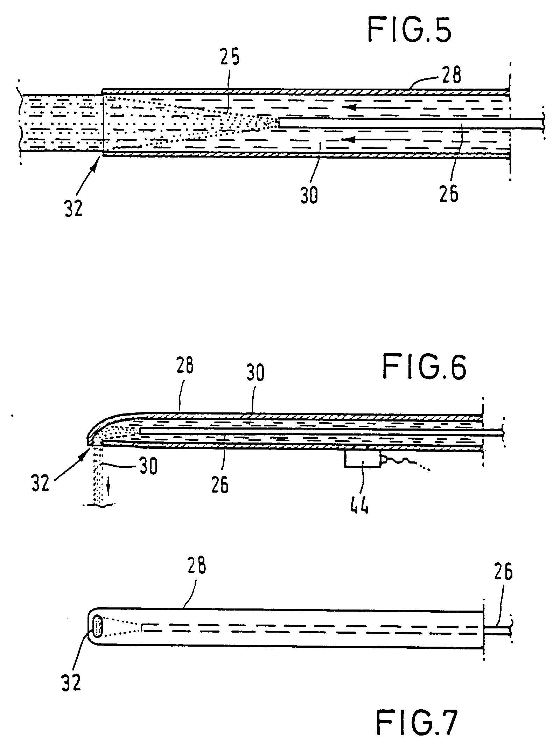

Die Entfernungen der Ablagerungen auf dem Zahn 2 erfolgt mit einer Vorrichtung, bestehend aus einer nicht dargestellten Laserlichtquelle, die einen gepulsten Laserlichtstrahl 25 über einen Lichtleiter 26 und einen Applikator 28 auf die mit Ablagerungen behaftete Zahnoberfläche leitet (Fig. 5). Der Applikator 28 besteht aus einem Handinstrument, dessen Spitze einen Durchmesser von ca. 1 bis 1,5 mm aufweist, um in die Zahnfleischtasche 6 eingeführt werden zu können.

Die Laserlichtquelle erzeugt einen gepulsten Laserlichtstrahl 25 mit einer Wellenlänge von 300 bis 600 nm und mit einer Energiedichte von 0,5 bis 10 J/cm². Ein Laserlichtstrahl mit diesen Parametern ist in der Lage selektiv die Ablagerungen 8,10,12 auf dem Zahn 2 zu entfernen, ohne die gesunde Zahnhartsubstanz, z.B. Schmelz unter supragingivalen Zahnstein 8 bzw. Schmelz, gesundes Wurzelzement und gesundes Wurzeldentin unter subgingivalem Zahnstein 8,12 zu schädigen. Des gleichen wird das Zahnfleischtaschengewebe und der angrenzende Zahnhalteapparat nicht verletzt. Das selektive Abtragen der Ablagerungen ist aufgrund des natürlichen Absorptionsverhaltens der Ablagerungen in dem genannten Wellenlängenbereich möglich.

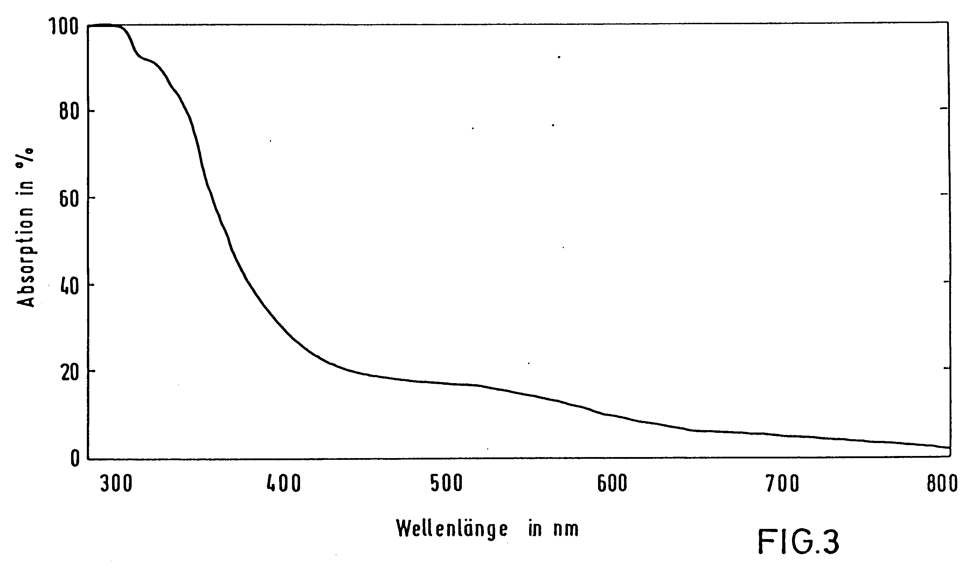

In der mikrobiellen Plaque 10 dominieren Bakterien. Fig. 3 zeigt das Absorptionsdiagramm des Bakterienstamms pseudomonas aerogenosa. Aufgetragen ist die Absorptionsintensität in Abhängigkeit von der Wellenlänge des Laserlichtes in nm. Oberhalb einer Wellenlänge von 600 nm ist die Absorption nur noch gering, während bei Wellenlängen unterhalb von 400 nm ein starker Anstieg der Absorption erkennbar ist. Dies ist auf die charakteristischen Absorptionsspektren aromatischer Aminosäuren sowie von Atmungskettenenzymen wie NAD, NADH, Cytochrom und Flavoproteinen etc. zurückzuführen. Typische Absorptionsspektra eines Flavinenzyms zeigen einen Absorptionsmaximum bei 370 und 480 nm. Bei Cytochrom C finden sich Absorptionsmaxima bei 400 nm und 550 nm.

Als parodontal pathogen werden insbesondere die Bakterienstämme porphyromonas gingivalis und prevotella intermedia angesehen, die auch unter dem Begriff schwarz-pigmentierte Bacteroides bekannt sind. Charakteristisch ist der Protoporphyrin IX (PPIX)-Gehalt. Protoporphyrin absorbiert Licht vorzugsweise bei 410 nm sowie bei 520 nm.

Der Anteil der Bakterien an der mikrobiellen Plaque 10 bedingt die möglicherweise etwas größere Absorption im nahen UV-Spektralbereich

Das Absorptionsverhalten von supragingivalem Zahnstein wird wesentlich durch die starke Präsenz von Bakterien beeinflußt und entspricht somit im wesentlichen dem Absorptionsverhalten von mikrobieller Placque 10. In mikrobiellen Plaque 10 sind allerdings nur wenige Blutbestandteile enthalten, die die Absorptionscharakteristik beeinflussen, aber die mikrobielle Plaque 10 und somit auch der supragingivale Zahnstein 8, als Form der mineralisierten Plaque, enthalten Eisen und Schwefel. Diese bilden unlösliches Eisensulfid, das schwarz gefärbt ist und somit Licht über den gesamten Spektralbereich absorbiert.

Für die Konkremente 12, nämlich dem subgingivalen Zahnstein, trägt der hohe Anteil an Einlagerungen von Blut und Blutbestandteilen zu einem Absorptionsverhalten bei, gemäß dem Absorptionsmaxima in dem Bereich zwischen 380 und 410 nm und bei 560 nm zu finden sind. Für die Konkremententfernung könnte daher auch ein Laser verwendet werden, der im sichtbaren Spektralbereich emittiert, z.B. ein kurzgepulster Argonlaser. Typischerweise sind derzeit allerdings noch ausschließlich kontinuierlich strahlende Laser erhältlich, die aufgrund thermischer Nebenwirkungen nicht einsetzbar sind.

Als Laserlichtquelle wird bevorzugt ein frequenzverdoppelter Alexandritlaser mit einer Wellenlänge von 380 nm oder ein frequenzverdreifachter Nd: YAG-Laser mit einer Wellenlänge von 355 nm verwendet.

Gute Ergebnisse lassen sich in dem Wellenlängenbereich zwischen 320 bis 410 nm mit einer Energiedichte von 1 bis 5 J/cm² erreichen. Bei Verwendung von Laserlicht mit einer Wellenlänge von 380 nm kann besonders vorteilhaft eine Energiedichte von ca. 3 J/cm² eingesetzt werden. Diese Parameterkombination gewährleistet, daß die Laserenergiedichte in bezug auf die genannte Wellenlänge unterhalb der Ablationsschwelle des gesunden Dentins 18 und damit auch unterhalb der Ablationsschwelle des gesunden Zahnschmelzes 5 liegt, die ihrerseits höher ist, als die von Dentin. Es ist somit möglich selektiv die auf dem Zahn 2 befindlichen Ablagerungen zu entfernen. Des weiteren wird die Wurzelzementschicht, soweit sie mit Bakterien infiziert ist, abgetragen. Dies ist insoweit wesentlich, als nur an nicht infiziertem Wurzelzement oder Dentinoberflächen Zementoblasten erneut hochwachsen, die den Zahnhalteapparat regenerieren.

Das Laserlicht ist des weiteren selektiv relativ zu dem Gewebe in der Zahnfleischtasche 6. Die Tascheninnenwand sowie der parodontale Halteapparat wird nicht nachteilig beeinflußt, wenn der Laserlichtstrahl auf die Taschenwand 21 bzw. auf den Taschenboden 22 trifft. Ein weiterer Vorteil der Entfernung der Ablagerung mit einem Laserlichtstrahl besteht auch darin, daß der Laserlichtstrahl die Bakterien in der Zahnfleischtasche 6 entweder unmittelbar zerstört oder ihren Stoffwechsel stört, so daß eine Desinfektion des gesamten Taschenbereiches stattfindet.

Die Laserlichtquelle emittiert vorzugsweise Laserimpulsketten mit gütegeschalteten Spikeimpulsen mit einer Spikefrequenz zwischen ca. 50 und 400 Spikes/s. Die Anzahl von Spikeimpulsen innerhalb einer Impulskette liegt im Bereich zwischen zwei und dreißig gütegeschalteten Spikeimpulsen. Der zeitliche Abstand zwischen den einzelnen Spikeimpulsen beträgt ca. 30 µs - 50 µs. Die Verwendung aufeinanderfolgender gütegeschalteter Spikeimpulse hat den Vorteil einer besseren Energienutzung sowie den Vorteil, höherer Frequenzen verwenden zu können, ohne hierzu einen größeren Transformator für die Laserlichtquelle zu benötigen.

Bei Bedarf ist die Impulslänge des Laserlichtstrahls 25 variabel einstellbar. Dabei sollte die Impulslänge 10 µs nicht überschreiten, da sonst eine thermische Schädigung des angrenzenden Gewebes nicht ausgeschlossen ist. Vorzugsweise wird eine Impulsdauer von ca. 200 ns verwendet. Hinsichtlich der Impulslänge des Laserlichtes über 200 ns kann ein Erhöhungsfaktor für die Energiedichte angewendet werden, der proportional zur Wurzel aus dem Quotienten der eingestellten Impulslänge und einer Basisimpulslänge von 200 ns ist. Vorzugsweise ist der Proportionalitätsfaktor 1. Dies bedeutet, daß bei Impulslängen über 200 ms eine entsprechend dem Erhöhungsfaktor höhere Energiedichte im Lichtpunkt benötigt wird.

Anstelle der Verwendung von Impulsketten aus mehreren gütegeschalteten Laserlichtspikes, ist es auch möglich, Impulsketten mit ungesteuert aufeinanderfolgenden Laserlichtspikes zu verwenden, wobei zwischen jeder Impulskette eine Dunkelphase von mehreren ms Dauer folgt.

Die zur selektiven Ablation der Ablagerungen verwendeten Laserlichtimpulse werden durch einen Lichtleiter 26 zu dem Applikator 28 geleitet.

Ab einer Pulsfrequenz von ca. 12 Hz sollte eine Kühlung des Arbeitspunktes im Lichtfleck des Laserlichtstrahls erfolgen. Hierzu kann eine bekannte Wasserspraykühlung zum Einsatz kommen, die an dem Applikator 28 angebracht sein kann.

Noch vorteilhafter ist es, den Laserlichtstrahl in dem Applikator 28 in einen Flüssigkeitsstrahl 30 einzukoppeln, wobei der Flüssigkeitsstrahl beim Austritt aus dem Applikator 28 als verlängerter Lichtleiter verwendet wird. Fig. 5 zeigt eine schematische Darstellung der Einkopplung des Laserlichtes in den Flüssigkeitsstrahl 30. Dabei liegt das freie Ende des Lichtleiters 26 relativ zur Austrittsöffnung 32 des Applikators 28 so weit zurück, daß unter Berücksichtigung des Öffnungswinkels des Laserlichtkegels der Laserlichtkegel an der Austrittsöffnung 32 des Applikators genau die Öffnungsweite der Austrittsöffnung 32 erreicht. Der Durchmesser des Applikators sollte maximal 1,0 bis 1,5 mm betragen. Bis zu diesem Durchmesser können Applikatoren verschiedener Durchmesser eingesetzt werden, um auch bequem in tiefe Zahnfleischtaschen 6 eindringen zu können. Hierzu könnte auch ein Applikator 28 mit verschiedenen austauschbaren Spitzen unterschiedlichen Durchmessers ausgerüstet werden.

Die Fign. 6 und 7 zeigen einen seitlich abstrahlenden Applikator, mit dem auch schwer zugängliche Bereiche, wie z.B. Wurzelfurkationen erreichbar sind. In den Applikatoren 28 können keramisch beschichtete Hohlleiter zum Einsatz kommen. Bei dem Ausführungsbeispiel der Fig. 7 ist eine elliptische Austrittsöffnung erkennbar, die eine flächige Bearbeitung der Zahnoberfläche ermöglicht.

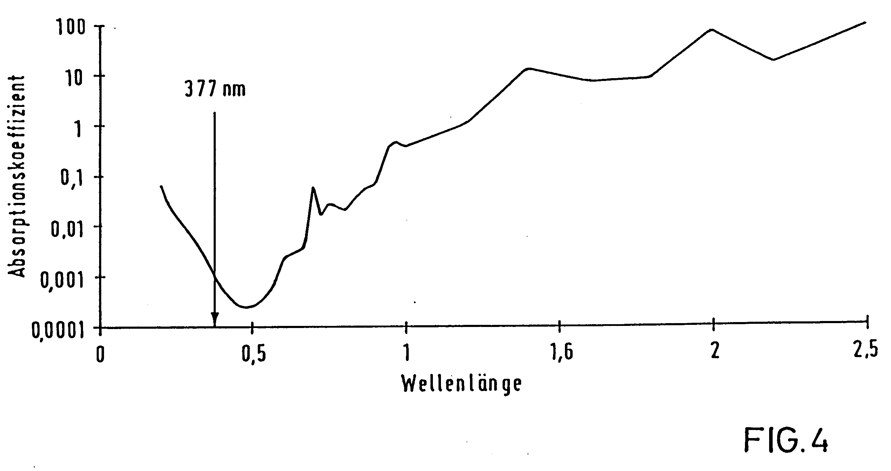

Die Wasserkühlung mit einem Flüssigkeitsstrahl 30, in den ein Laserlichtstrahl 25 eingekoppelt ist, hat den Vorteil, daß die Kühlung genau in dem Arbeitspunkt des Laserlichtstrahls erfolgt. Vorteilhaft ist dabei, daß wie aus dem in Fig. 4 dargestellten Absorptionsspektrum für Wasser ersichtlich ist, in dem verwendeten Wellenlängenbereich praktisch keine Absorption des Laserlichtes durch Wasser erfolgt. Insofern wird die Ablation durch das in der Zahnfleischtasche 6 befindliche Wasser nicht beeinträchtigt.

Die Kontrolle der Ablation kann fiberoptisch mit Hilfe eines optischen Kontrollinstrumentes 34 erfolgen. Das optische Kontrollinstrument 34 ist ein separates Handinstrument, das unabhängig von dem Applikator 28 benutzt werden kann und beispielsweise in Fig. 1 bei der Inspektion der Zahnfleischtasche 6 dargestellt ist.

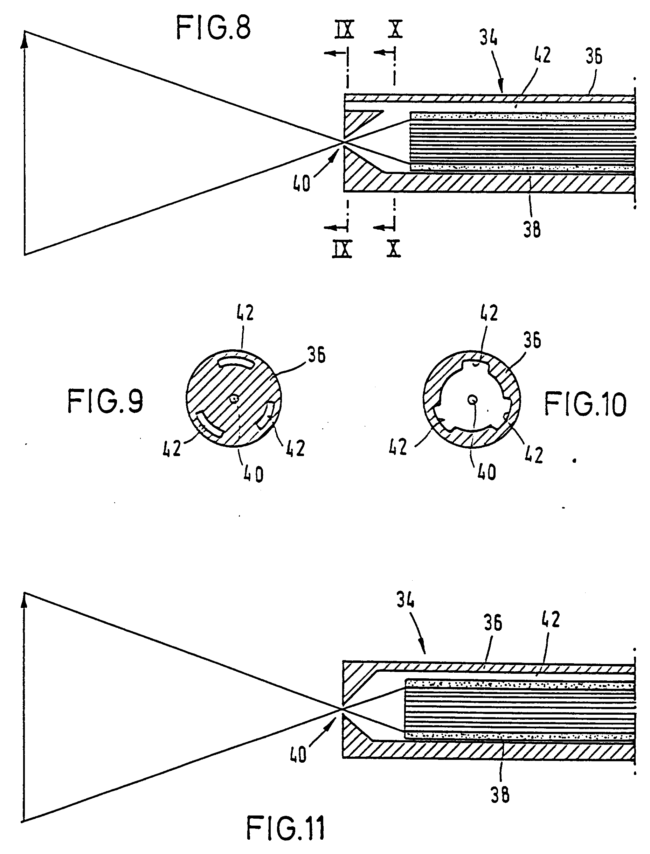

Fig. 8 zeigt ein erstes Ausführungsbeispiel eines optischen Kontrollinstrumentes 34, bestehend aus einem nicht dargestellten optischen Sensor, der über einen Bildlichtleiter 38 mit dem Handstück 36 verbunden ist. Der Bildlichtleiter 38 besteht aus einem Bündel von Lichtleitern und bewirkt im Vergleich zu einem einzigen Lichtleiter eine Übertragung einer Bildinformation. Der Bildlichtleiter 38 endet mit Abstand von einer Lochblende 40, mit deren Hilfe ein Bild der Zahnfleischtasche 6 auf dem Bildlichtleiter 38 erzeugt wird. Das Handstück 36 weist ferner mindestens einen Kanal 42 für eine Spülflüssigkeit auf, die entweder bei den Ausführungsbeispielen der Fign. 8 bis 10 im Umfangsbereich der Lochblende 40 und aus der Lochblende 40 selber aus dem Handstück 36 austritt oder, wie in dem Ausführungsbeispiel der Fig. 11 gezeigt, nur durch die Lochblende 40 selbst austritt. Die Bildübertragung wird durch den Wasseraustritt begünstigt, da die Zahnfleischtasche 6 ohnehin mit Flüssigkeit gefüllt ist. Aufgrund des Laserlichtes des Applikators ist die Zahnoberfläche mit hoher Lichtintensität beleuchtet, so daß die Lochblende ein scharfes und helles Bild von der Zahnoberfläche erzeugen kann.

Eine weitere Möglichkeit den Arbeitsfortschritt und den Arbeitserfolg zu kontrollieren, besteht darin, den Applikator 28 mit einem akustischen Sensor zu versehen, der die Ablationsgeräusche aufnimmt. Sofern sich noch Ablagerungen auf der Zahnoberfläche befinden, werden bei der Ablation infolge des Ablationsdrucks Geräusche erzeugt, während bei einer gereinigten Zahnoberfläche keine Geräusche mehr feststellbar sind. Besonders vorteilhaft ist dabei, bei einem Applikator gemäß den Ausführungsbeispielen der Fign. 5 bis 7, den rücklaufenden Körperschall in dem Flüssigkeitsstrahl 30 mit einem akustischen Sensor 44 zu detektieren, der das Ablationsgeräusch über den Körperschall am besten erfassen kann.

## Patentansprüche

1. Vorrichtung zum Entfernen von Ablagerungen, wie z.B. Zahnstein (8), mikrobieller Plaque (10) und Konkrementen (12) auf Zähnen (2) mit einer Laserlichtquelle mit einem bis zu einem Applikator (28) geführten Lichtleiter (26) für einen gepulsten Laserlichtstrahl (25), wobei
die Wellenlänge der Laserlichtquelle auf einen Wert im Bereich von 300 bis 600 nm, vorzugsweise 320 bis 410 nm, derart entsprechend dem Absorptionsverhalten der Ablagerungen und von bakteriell infiziertem Wurzelzement abgestimmt ist, daß die genannten Substanzen selektiv entfernt werden, und wobei
die Laserlichtquelle eine Energiedichte von 0,5 bis 10 J/cm² im Lichtfleck aufweist, **dadurch gekennzeichnet,**
daß der Laserlichtstrahl (25) in einen Flüssigkeitsstrahl (30) eingekoppelt ist und koaxial mit dem Flüssigkeitsstrahl (30) aus dem Applikator (28) austritt, und
daß der Applikator (28) aus einem Handinstrument besteht, dessen Spitze einen Durchmesser von ca. 1 bis 1,5 mm aufweist.

2. Vorrichtung nach Anspruch 1, dadurch gekennzeichnet, daß die Pulsdauer eines Laserlichtimpulses des Laserlichtstrahls (25) oder eines Spikeimpulses einer Impulskette eines Laserlichtimpulses weniger als 10 µs, vorzugsweise zwischen 5 ns und 5 µs, beträgt.

3. Vorrichtung nach einem der Ansprüche 1 oder 2, dadurch gekennzeichnet, daß die Pulsdauer des Laserlichtstrahls (25) zwischen 50 und 300 ns, vorzugsweise zwischen 150 und 250 ns, beträgt.

4. Vorrichtung nach einem der Ansprüche 1 bis 3, dadurch gekennzeichnet, daß die Pulswiederholungsfrequenz der Laserlichtquelle zwischen 10 und 200 Hz, vorzugsweise zwischen 15 und 150 Hz, beträgt.

5. Vorrichtung nach einem der Ansprüche 1 bis 4, dadurch gekennzeichnet, daß eine Impulskette eines Laserlichtimpulses zwei bis 30 gütegeschaltete Spikeimpulse enthält.

6. Vorrichtung nach Anspruch 5, dadurch gekennzeichnet, daß jeder Laserlichtimpuls zwei Spikeimpulse enthält.

7. Vorrichtung nach Anspruch 5 oder 6, dadurch gekennzeichnet, daß die Doppel- oder Mehrfachspikes einen zeitlichen Abstand von ca. 30 µs haben.

8. Vorrichtung nach einem der Ansprüche 1 bis 4, dadurch gekennzeichnet, daß Impulsketten aus mehreren Laserlichtspikes ungesteuert aufeinanderfolgen (free running laser), und daß zwischen jeder Impulskette eine Dunkelphase von mehreren ms Dauer folgt.

9. Vorrichtung nach einem der Ansprüche 1 bis 8, dadurch gekennzeichnet, daß das Laserlicht des Laserlichtstrahl (25) eine Energiedichte im Lichtfleck zwischen 0,5 und 5 J/cm² aufweist.

10. Vorrichtung nach einem der Ansprüche 1 bis 9, dadurch gekennzeichnet, daß das Laserlicht des Laserlichtstrahls (25) eine Wellenlänge zwischen 355 und 390 nm besitzt.

11. Vorrichtung nach einem der Ansprüche 1 bis 10, dadurch gekennzeichnet, daß die Energiedichte des Laserlichtstrahls (25) im Lichtfleck zwischen 0,6 und 3,6 J/cm² beträgt.

12. Vorrichtung nach einem der Ansprüche 1 bis 11, dadurch gekennzeichnet, daß der Applikator (28) an dem freien Ende eine seitlich zur Längsachse des Applikators (28) verlaufende Austrittsöffnung (32) für den Laserlichtstrahl (25) aufweist.

13. Vorrichtung nach einem der Ansprüche 1 bis 12, dadurch gekennzeichnet, daß an dem Applikator (28) ein akustischer Sensor (44) derart angeordnet ist, daß Ablationsgeräusche zur Anzeige des Arbeitsfortschritts meßbar sind.

14. Vorrichtung nach einem der Ansprüche 1 bis 13, dadurch gekennzeichnet, daß eine Kontrolleinrichtung (34) mit einen optischen Sensor an dem Applikator (28) oder in einem separaten Handstück (36) angeordnet ist.

15. Vorrichtung nach Anspruch 14, dadurch gekennzeichnet, daß dem optischen Sensor eine Lochblende (40) vorgeschaltet ist.

16. Vorrichtung nach Anspruch 15, dadurch gekennzeichnet, daß zwischen optischem Sensor und Lochblende (40) ein Bildlichtleiter (38) zwischengeschaltet ist.

17. Vorrichtung nach einem der Ansprüche 14 oder 15, dadurch gekennzeichnet, daß eine Spülflüssigkeit durch die Lochblende (40) des optischen Sensors hindurchtritt und die Bildübertragung durch die Lochblende (40) hindurch unterstützt.

18. Vorrichtung nach einem der Ansprüche 1 bis 16, dadurch gekennzeichnet, daß der laserlichtleitende Flüssigkeitsstrahl (30) entgegen der Durchflußrichtung der Flüssigkeit den Körperschall als akustisches Signal an einen akustischen Sensor überträgt.

## Claims

1. A device for the removal of deposits, e.g. tartar (8), microbial plaque (10) and concrements (12) on a tooth (2), comprising a laser light source for a pulsed laser light beam (25), said laser light source being provided with a light conductor (26) leading to an applicator (28), wherein
the wavelength of said laser light source is adjusted to a value in the range from 300 to 600 nm, preferably from 320 to 410 nm, in correspondence to absorption behavior of said deposits and of root cement infected with bacteria, in such a manner that said substances are selectively removed, and wherein
the laser light source has an energy density of 0.5 to 10 J/cm² in the light spot,
**characterized in**
that the laser light beam (25) is enclosed in a liquid jet (30) and is discharged from the applicator (28) coaxially with said liquid jet (30), and
that the applicator (28) comprises a hand-held instrument of which the tip has a diameter of about 1 to 1.5 mm.

2. The device according to claim 1, characterized in that the pulse length of a laser light pulse of the laser light beam (25) or of a spike pulse of a pulse chain of a laser light pulse is less than 10 µs, and preferably is between 5 ns and 5 µs.

3. The device according to claim 1 or 2, characterized in that the pulse length of the laser light beam (25) is between 50 and 300 ns, preferably between 150 and 250 ns.

4. The device according to any one of claims 1 to 3, characterized in that the pulse repetition frequency of the laser light source is between 10 and 200 Hz, and preferably is between 15 and 150 Hz.

5. The device according to any one of claims 1 to 4, characterized in that a pulse chain of a laser light pulse contains two to 30 Q-switched spike pulses.

6. The device according to claim 5, characterized in that each laser light pulse contains two spike pulses.

7. The device according to claim 5 or 6, characterized in that the distance in time between the double or multiple spike pulses is about 30 µs.

8. The device according to any one of claims 1 to 4, characterized in that pulse chains comprising a plurality of laser light spikes follow each other in an uncontrolled manner (free running laser), and that each pulse chain is followed by a dark phase of a duration of several ms.

9. The device according to any one of claims 1 to 8, characterized in that the laser light of the laser light beam (25) has an energy density in the light spot between 0.5 and 5 J/cm².

10. The device according to any one of claims 1 to 9, characterized in that the laser light of the laser light beam (25) has a wavelength between 355 and 390 nm.

11. The device according to any one of claims 1 to 10, characterized in that the energy density of the laser light beam (25) in the light spot is between 0.6 and 3,6 J/cm².

12. The device according to any one of claims 1 to 11, characterized in that the applicator (28) has its free end formed with an outlet opening (32) for the laser light beam (25) extending laterally of the longitudinal axis of the applicator (28).

13. The device according to any one of claims 1 to 12, characterized in that the applicator (28) has an acoustic sensor (44) arranged thereon in a manner allowing measurement of ablation noises indicating the progress of the operation.

14. The device according to any one of claims 1 to 13, characterized in that a monitoring means (34) comprising an optical sensor is arranged on the applicator (28) or in a separate hand-held piece (36).

15. The device according to claim 14, characterized in that an aperture plate (40) is arranged before said optical sensor.

16. The device according to claim 15, characterized in that an image light conductor (38) is connected between the optical sensor and the aperture plate (40).

17. The device according to claim 14 or 15, characterized in that a rinsing liquid passes through the aperture plate (40) of the optical sensor to enhance the image transmission through the aperture plate (40).

18. The device according to any one of claims 1 to 16, characterized in that the laser light conducting liquid jet (30) transmits the body sound as an acoustic signal to an acoustic sensor in the opposite sense to the flow direction of the liquid.

## Revendications

1. Dispositif d'élimination de dépôts, comme par exemple le tartre (8), la plaque microbienne (10) et des concrétions (12) sur les dents (2) au moyen d'une source de lumière laser avec une fibre optique (26) menée jusqu' à un applicateur (28) pour un faisceau de lumière laser pulsé (25), dispositif dans lequel la longueur d'onde de la source de lumière laser est réglée à une valeur dans la zone de 300 à 600 nm, de préférence de 320 à 410 nm, de telle sorte que, selon le comportement quant à l'absorption des dépôts et du cément de la racine infecté par des bactéries, lesdites substances sont éliminées sélectivement, et dans lequel la source de lumière laser présente une intensité énergétique de 0,5 à 10 J/cm² dans la tache de lumière,
caractérisé en ce que le faisceau de lumière laser (25) est couplé à un jet de liquide (30) et sort de l'applicateur (28) coaxialement au jet de liquide (30), et
en ce que l'applicateur (28) se compose d'un instrument à main dont l'extrémité présente un diamètre d'environ 1 à 1,5 mm .

2. Dispositif selon la revendication 1, caractérisé en ce que la durée d'impulsion d'une impulsion de lumière laser du faisceau de lumière laser (25) ou d'une impulsion de spike d'un train d'impulsions d'une impulsion de lumière laser est inférieure à 10 µs, de préférence est comprise entre 5 ns et 5 µs.

3. Dispositif selon la revendication 1 ou 2, caractérisé en ce que la durée d'impulsion du faisceau de lumière laser (25) est comprise entre 50 et 300 ns, de préférence entre 150 et 250 ns.

4. Dispositif selon l'une des revendications 1 à 3, caractérisé en ce que la fréquence de répétition des impulsions de la source de lumière laser est comprise entre 10 et 200 Hz, de préférence entre 15 et 150 Hz.

5. Dispositif selon l'une des revendications 1 à 4, caractérisé en ce qu'un train d'impulsions d'une impulsion de lumière laser comprend deux à 30 impulsions de spike déclenchées.

6. Dispositif selon la revendication 5, caractérisé en ce que chaque impulsion de lumière laser contient deux impulsions de spike.

7. Dispositif selon la revendication 5 ou 6, caractérisé en ce que les spikes doubles ou multiples sont temporellement distants d'environ 30 µs .

8. Dispositif selon l'une des revendications 1 à 4, caractérisé en ce que des trains d'impulsions constitués de plusieurs spikes de lumière laser se suivent sans commande (free running laser) et en ce qu'une phase d'extinction d'une durée de plusieurs ms fait suite entre chaque train d'impulsions.

9. Dispositif selon l'une des revendications 1 à 8, caractérisé en ce que la lumière laser du faisceau de lumière laser (25) a une intensité énergétique dans la tache de lumière comprise entre 0,5 et 5 J/cm².

10. Dispositif selon l'une des revendications 1 à 9, caractérisé en ce que la lumière laser du faisceau de lumière laser (25) a une longueur d'onde comprise entre 355 et 390 nm.

11. Dispositif selon l'une des revendications 1 à 10, caractérisé en ce que l'intensité énergétique du faisceau de lumière laser (25) dans la tache de lumière est comprise entre 0,6 et 3,6 J/cm².

12. Dispositif selon l'une des revendications 1 à 11, caractérisé en ce que l'applicateur (28) présente à son extrémité libre une ouverture de sortie (32) pour le faisceau de lumière laser (25) qui s'étend latéralement par rapport à l'axe longitudinal de l'applicateur (28).

13. Dispositif selon l'une des revendications 1 à 12, caractérisé en ce qu'un capteur acoustique (44) est disposé sur l'applicateur (28) de telle sorte que les bruits engendrés par l'ablation Puissent être mesurés pour une indication de l'avancement du travail.

14. Dispositif selon l'une des revendications 1 à 13, caractérisé en ce qu'un dispositif de contrôle (34) comportant un capteur optique est disposé sur l'applicateur (28) ou dans une pièce à main séparée (36).

15. Dispositif selon la revendication 14, caractérisé en ce qu'un sténopé (40) est disposé à l'avant du capteur optique.

16. Dispositif selon la revendication 15, caractérisé en ce qu'un guide d'image (38) est disposé entre le capteur optique et le sténopé (40).

17. Dispositif selon la revendication 14 ou 15, caractérisé en ce qu'un liquide de rinçage passe à travers le sténopé (40) du capteur optique et en ce que la transmission d'image est assistée par le sténopé (40).

18. Dispositif selon l'une des revendications 1 à 16, caractérisé en ce que le jet de liquide (30) conduisant la lumière laser transmet à un capteur acoustique, dans le sens contraire à celui de l'écoulement du liquide, le bruit du corps comme signal acoustique.
